# EUROPÄISCHE PATENTANMELDUNG

(11) **EP 3 248 662 A1**
(43) Veröffentlichungstag der Anmeldung: **29.11.2017**
(21) Anmeldenummer: 16171093.4
(22) Anmeldetag: 24.05.2016
(51) Int. Cl.: B01D 3/00, C07C 2/84, C07C 5/48, C02F 1/04

(54) **VERFAHREN ZUR GEWINNUNG VON TRINK-, BEWÄSSERUNGS- UND/ODER BETRIEBSWASSER**

(71) Anmelder: Linde Aktiengesellschaft, 80331 München (DE)
(72) Erfinder: Zimmermann, Heinz, 81476 München (DE); Fritz, Helmut, 81375 München (DE); Haidegger, Ernst, 85521 Riemerling (DE); Kemper, Rainer, 81375 München (DE)
(74) Vertreter: m patent group

(57) **Zusammenfassung**

Die Erfindung betrifft ein Verfahren (100) zur Gewinnung von Trink-, Bewässerungs- und/oder Betriebswasser mittels eines oder mehrerer Aufbereitungsschritte (20, 30). Es ist vorgesehen, dass ein Methankopplungschritt (10) durchgeführt wird, in dem Methan mit Sauerstoff durch oxidative Kopplung von Methan (OCM) unter Bildung von Reaktionswasser und Freisetzung von Reaktionswärme katalytisch umgesetzt wird, und dass zumindest ein Teil des Reaktionswassers und/oder zumindest ein Teil der Reaktionswärme in dem oder den Aufbereitungsschritten (20, 30) eingesetzt wird. Eine entsprechende Anlage ist ebenfalls Gegenstand der vorliegenden Erfindung.

## Beschreibung

Die vorliegende Erfindung betrifft ein Verfahren zur Gewinnung von Trink-, Bewässerungs- und/oder Betriebswasser mittels eines oder mehrerer Aufbereitungsschritte sowie eine entsprechende Anlage gemäß den Oberbegriffen der jeweiligen unabhängigen Patentansprüche.

### Stand der Technik

Insbesondere in den Ländern des nahen Ostens kommt zur Gewinnung von Trink-, Bewässerungs- und/oder Betriebswasser die Meerwasserentsalzung zum Einsatz. Herkömmlicherweise ist die Meerwasserentsalzung ausgesprochen energieintensiv. Als Energieträger werden typischerweise fossile Rohstoffe wie Schweröl oder Erdgas eingesetzt. Auch neuere, bei der Meerwasserentsalzung verwendete Verfahren wie die Umkehrosmose besitzen einen hohen Energieverbrauch und durch die Salzgehalte des Meerwassers nur ca. 30 bis 40% Ausbeute. Die Kosten pro Kubikmeter entsalzten Meerwassers werden dabei mit typischerweise 1,50 bis 1,70 US-Dollar angenommen.

Neben dem hohen Energieaufwand und der schlechten Ausbeute bei der Entsalzung von Meerwasser kann auch der vergleichsweise hohe Gehalt an Bor und ggf. anderen Spurenelementen im Meerwasser Probleme bereiten, insbesondere, wenn durch Meerwasserentsalzung Bewässerungswasser für die Landwirtschaft gewonnen werden soll. Bor kann sich in Böden anreichern und hat insbesondere in höheren Konzentrationen negative Effekte auf das Pflanzenwachstum.

Ist nachfolgend von "Trink-, Bewässerungs- und/oder Betriebswasser" die Rede, sei hierunter sowohl Wasser für den Bedarf der menschlichen Ernährung, als auch Wasser zum Einsatz in der Landwirtschaft, beispielsweise zur Bewässerung von Feldern, verstanden. Für die Reinheit von Trink- und Bewässerungswasser gelten jeweils Grenzwerte, die der Fachmann bei der Meerwasserentsalzung und ggf. dieser nachgeschalteten Aufbereitungsschritten berücksichtigt. Unter Betriebswasser wird im Rahmen dieser Anmeldung beispielsweise Kühl- und/oder Kesselspeisewasser verstanden, beispielsweise wie für kalorische Kraftwerke.

Vor dem erläuterten Hintergrund besteht der Bedarf, Verfahren zur Gewinnung von Trink-, Bewässerungs- und/oder Betriebswasser zu verbessern und insbesondere energieeffizienter auszugestalten.

### Offenbarung der Erfindung

Vor diesem Hintergrund schlägt die vorliegende Erfindung ein Verfahren zur Gewinnung von Trink-, Bewässerungs- und/oder Betriebswasser mittels eines oder mehrerer Aufbereitungsschritte sowie ein entsprechendes Verfahren mit den Merkmalen der jeweiligen unabhängigen Patentansprüche vor. Bevorzugte Ausgestaltung sind jeweils Gegenstand der abhängigen Patentansprüche sowie der nachfolgenden Beschreibung.

### Vorteile der Erfindung

Die vorliegende Erfindung beruht auf der Erkenntnis, dass die oxidative Kopplung von Methan in Kombination mit der Gewinnung von Trink-, Bewässerungs- und/oder Betriebswasser besondere Vorteile bietet.

Die Herstellung höherer Kohlenwasserstoffe aus Methan durch oxidative Methankopplung (engl. Oxidative Coupling of Methane, OCM) befindet sich derzeit in intensiver Entwicklung. Bei der oxidativen Methankopplung handelt es sich um die direkte Umsetzung von Methan in einem oxidativen, heterogen katalysierten Verfahren zu höheren Kohlenwasserstoffen. Entsprechende Verfahren erscheinen insbesondere zur Herstellung von Ethylen vielversprechend.

Bezüglich weiterer Details der oxidativen Methankopplung sei auf einschlägige Fachliteratur, beispielsweise Zavyalova, U. et al.: Statistical Analysis of Past Catalytic Data on Oxidative Methane Coupling for New Insights into the Composition of High-Performance Catalysts, ChemCatChem 3, 2011, 1935-1947, verwiesen.

Bei der oxidativen Methankopplung wird ein methanreicher Strom, beispielsweise Erdgas oder ein aus Erdgas gebildeter methanreicher Strom, zusammen mit einem sauerstoffreichen Stoffstrom einem Reaktor zugeführt. Es wird ein Produktstrom erhalten, der neben Reaktionsprodukten der oxidativen Methankopplung, insbesondere Ethylen, ggf. Propylen und höheren Kohlenwasserstoffen, auch Wasserstoff und Kohlendioxid, nicht umgesetztes Methan und nicht umgesetzten Sauerstoff enthält. Wird beispielsweise stickstoffhaltiges Erdgas bei der oxidativen Methankopplung eingesetzt, enthält der Produktstrom auch Stickstoff.

Die oxidative Kopplung von Methan bietet die Möglichkeit, Methan, beispielsweise aus Erdgas, alternativ stofflich zu nutzen und nicht mehr nur, wie derzeit, überwiegend zu verfeuern oder zu Synthesegas umzusetzen. Dies ist insbesondere im Hinblick auf schwindende Ressourcen fossiler Energieträger von großer Bedeutung.

Die vorliegende Erfindung schlägt dabei ein Verfahren zur Gewinnung von Trink-, Bewässerungs- und/oder Betriebswasser mittels eines oder mehrerer Aufbereitungsschritte vor. Wie nachfolgend erläutert, können entsprechende Aufbereitungsschritte insbesondere einen oder mehrere Meerwasserentsalzungsschritte umfassen, jedoch auch alternativ oder zusätzlich einen oder mehrere Aufbereitungsschritte, bei dem oder denen Wasser, ohne es zu entsalzen, aufgereinigt wird. Derartige Schritte können beispielsweise Strippen, Aktivkohleadsorption und/oder Umkehrosmose umfassen. Je nach Art der beabsichtigten Verwendung kann beispielsweise auch eine Entkeimung, Remineralisierung und dergleichen vorgesehen sein.

Erfindungsgemäß ist vorgesehen, einen Methankopplungsschritt durchzuführen, in dem Methan mit Sauerstoff unter Bildung von Reaktionswasser und unter Freisetzung von Reaktionswärme katalytisch umgesetzt wird. Im Rahmen der vorliegenden Erfindung wird also ein zuvor erläutertes Verfahren zur oxidativen Methankopplung durchgeführt. Erfindungsgemäß ist ferner vorgesehen, dass zumindest ein Teil des Reaktionswassers aus dem Methankopplungsschritt und/oder zumindest einen Teil der in dem Methankopplungsschritt gebildeten Reaktionswärme in dem oder den Aufbereitungsschritten zur Gewinnung des Trink-, Bewässerungs- und/oder Betriebswassers eingesetzt wird.

Derzeit durchgeführte Methankopplungsschritte der zuvor erläuterten Art führen zur Bildung von ca. zwei Kubikmeter Wasser pro Tonne Ethylen. Dieses Wasser ist zwar mit und ggf. Nebenprodukten aus der Methankopplung verunreinigt, kann aber durch entsprechende Verfahren, wie nachfolgend erläutert, kostengünstig zu Trink-, Bewässerungs- und/oder Betriebswasser aufbereitet werden. Die vorliegende Erfindung kann jedoch auch eine weitere Integration zwischen oxidativer Methankopplung und Wassergewinnung umfassen, wie nachfolgend erläutert.

Vorteilhafterweise werden im Rahmen des erfindungsgemäßen Verfahrens mehrere Aufbereitungsschritte zur Gewinnung des Trink-, Bewässerungs- und/oder Betriebswassers durchgeführt. Diese Schritte umfassen vorzugsweise einen ersten Aufbereitungsschritt und einen zweiten Aufbereitungsschritt. Der erste und der zweite Aufbereitungsschritt sind vorzugsweise sequentiell angeordnet, so dass zumindest ein Teil bei der Gewinnung des Trink-, Bewässerungs- und/oder Betriebswassers verwendeten Einsatzwassers beide Aufbereitungsschritte durchläuft.

Von besonderem Vorteil ist es dabei, wenn im ersten Aufbereitungsschritt Meerwasser unter Einsatz der Reaktionswärme des Methankopplungsschritts entsalzt wird. Ist hier davon die Rede, dass das Meerwasser "unter Einsatz" entsprechender Reaktionswärme entsalzt wird, sei hierunter verstanden, die gesamte oder ein Teil der Reaktionswärme bei der Entsalzung des Meerwassers genutzt wird. Dabei können bei der Entsalzung des Meerwassers auch weitere Energiequellen zusätzlich verwendet werden. Beispielsweise kann im Rahmen der vorliegenden Erfindung zusätzlich Methan und/oder Schweröl verbrannt werden, um beispielsweise Niederdruckdampf zu produzieren. In jedem Fall verringert die vorliegende Erfindung jedoch die für die Meerwasserentsalzung direkt benötigte Energie signifikant. Die Reaktionswärme des Methankopplungsschritts kann insbesondere über geeignete Wärmeüberträgermedien wie Heißdampf in den ersten Aufbereitungsschritt übertragen werden.

Besondere Vorteile ergeben sich dabei, wenn die Entsalzung in dem ersten Aufbereitungsschritt eine gestufte Destillation bei reduziertem Druck (engl. Multiple Effect Distillation, MED) umfasst. Bei der gestuften Destillation bei reduziertem Druck handelt es sich um einen Destillationsprozess auf mehreren Stufen. In jeder Stufe wird Einsatzwasser mittels Dampf erhitzt. Auf jeder Stufe verdampft ein Teil des Wassers und fließt zur nächsten Stufe, wo auch dieser Dampf zur Verdampfung beiträgt. Jede Stufe nutzt damit, mit anderen Worten, die Energie der vorherigen Stufe. Im Rahmen der gestuften Destillation bei reduziertem Druck kann insbesondere Niederdruckdampf zum Einsatz kommen, wie er sich unter Verwendung der Reaktionswärme des Methankopplungsschritts auf besonders günstige Weise bilden lässt. In einem Abstrom eines für den Methankopplungsschritt verwendeten Reaktors kann dabei beispielsweise eine Dampferzeugungseinheit vorgesehen sein, die auch beispielsweise mit im Rahmen des erfindungsgemäßen Verfahrens entsalztem Wasser und/oder Reaktionswasser gespeist werden kann.

Als besonders vorteilhaft erweist es sich, wenn in einem entsprechenden, mindestens zwei Aufbereitungsschritte umfassenden Verfahren das Meerwasser, das zuvor in dem ersten Aufbereitungsschritt entsalzt wurde, in dem zweiten Aufbereitungsschritt nachbehandelt wird. Zur Nachbehandlung können bekannte Verfahren zum Einsatz kommen, wie sie nachfolgend erläutert werden. Der zweite Aufbereitungsschritt dient beispielsweise zur Entfernung von Restverunreinigungen aus dem Meerwasser.

Vorteilhafterweise kann der erste und/oder zweite Aufbereitungsschritt auch zur Aufreinigung des Reaktionswassers oder jeweils eines Teils hiervon genutzt werden. Der zweite Aufbereitungsschritt kann daher beispielsweise mit Einsatzwasser gespeist werden, das sowohl aus der Meerwasserentsalzung als auch aus dem Reaktionswasser des Methankopplungsschritts stammt. Ist eine Aufbereitung des Reaktionswassers erforderlich, die verfahrenstechnisch einer Entsalzung ähnelt, kann das Reaktionswasser auch der Entsalzung oder einem vergleichbaren Schritt, also beispielsweise dem ersten Aufbereitungsschritt der vorliegenden Erfindung, zugeführt werden. Das Reaktionswasser kann auch zuvor geeigneten Vorbehandlungsschritten unterworfen werden.

Wie bereits zuvor erläutert, ist das erfindungsgemäße Verfahren jedoch nicht zwingend im Zusammenhang mit einer Meerwasserentsalzung ausgestaltet. So können der oder die verwendeten Aufbereitungsschritte auch lediglich einen Nebenproduktentfernungsschritt umfassen, dem das Reaktionswasser zugeführt wird. In dem Nebenproduktentfernungsschritt wird oder werden Nebenprodukte der Methankopplung, die für den jeweiligen Verwendungszweck stören, entfernt. Das Verfahren in dieser Ausführungsform stellt damit Trink-, Bewässerungs- und/oder Betriebswasser ausschließlich aus dem Reaktionswasser des Methankopplungsschritts bereit und es muss keine zusätzliche Meerwasserentsalzung durchgeführt werden. In dieser Verfahrensvariante kann die Reaktionswärme des Methankopplungsschritts für andere Zwecke genutzt werden. Alternativ kann dem oder den Aufbereitungsschritten auch Wasser aus anderen Quellen, beispielsweise aus anderen chemischen Prozessen oder aus einer Abwasserbehandlungsanlage, zugeführt und dort gereinigt werden. Auch diese Reinigung kann zusammen mit dem Reaktionswasser des Methankopplungsschritts erfolgen.

Mit besonderem Vorteil können der zweite Aufbereitungsschritt und/oder der Nebenproduktentfernungsschritt einen adsorptiven, einen desorptiven und/oder einen osmotischen Reinigungsschritt umfassen. Beispielsweise können Restverunreinigungen aus dem entsalzten Wasser und/oder Kohlenwasserstoffe aus dem Reaktionswasser mittels Aktivkohle entfernt werden. Auch Verfahren zur Umkehrosmose können im Rahmen der vorliegenden Erfindung eingesetzt werden. Durch entsprechende Schritte wird eine spezifikationsgerechte Gewinnung von Trink-, Bewässerungs- und/oder Betriebswasser sichergestellt. Wie erwähnt, kann auch beispielsweise eine Remineralisierung und/oder eine Entkeimung, insbesondere für die Gewinnung von Trinkwasser, erfolgen.

Die vorliegende Erfindung erstreckt sich auch auf eine Anlage zur Gewinnung von Trink-, Bewässerungs- und/oder Betriebswasser mit einer oder mehrerer Aufbereitungseinheiten. Eine derartige Anlage zeichnet sich durch eine Methankopplungseinheit, umfassend einen oder mehrerer geeigneter Reaktoren, aus, die dafür eingerichtet ist, Methan mit Sauerstoff unter Bildung von Reaktionswasser und Freisetzung von Reaktionswärme umzusetzen. Eine derartige Methankopplungseinheit ist beispielsweise dadurch für eine Methankopplung eingerichtet, dass sie ein geeignetes Katalysatorbett mit einem spezifisch ausgewählten Katalysator umfasst und bei entsprechenden Drücken und Temperaturen betrieben werden kann. Ferner zeichnet sich eine entsprechende Anlage durch Mittel aus, die dafür eingerichtet sind, zumindest einen Teil des Reaktionswassers und/oder zumindest einen Teil der Reaktionswärme der oder den Aufbereitungseinheiten zuzuführen.

Insbesondere umfasst eine derartige Anlage eine Niederdruckdampferzeugungseinheit, die dafür eingerichtet ist, unter Verwendung der Reaktionswärme Dampf zu erzeugen und den Dampf der oder den Aufbereitungseinheiten als Energieträger zuzuführen, wie zuvor erläutert. Die Niederdruckdampferzeugungseinheit kann als Teil eines Dampfsystems ausgeführt sein, in dem beispielsweise auch Wasser, Dampf oder Wärme aus anderen Anlagenteilen verwendet werden kann.

Eine entsprechende Anlage ist vorteilhafterweise zur Durchführung eines Verfahrens eingerichtet, wie es zuvor erläutert wurde. Auf die jeweils erwähnten Merkmale und Vorteile sei daher an dieser Stelle ausdrücklich verwiesen.

Die Erfindung wird nachfolgend unter Bezugnahme auf die beigefügte Zeichnung näher erläutert, in der eine bevorzugte Ausführungsform der Erfindung gezeigt ist.

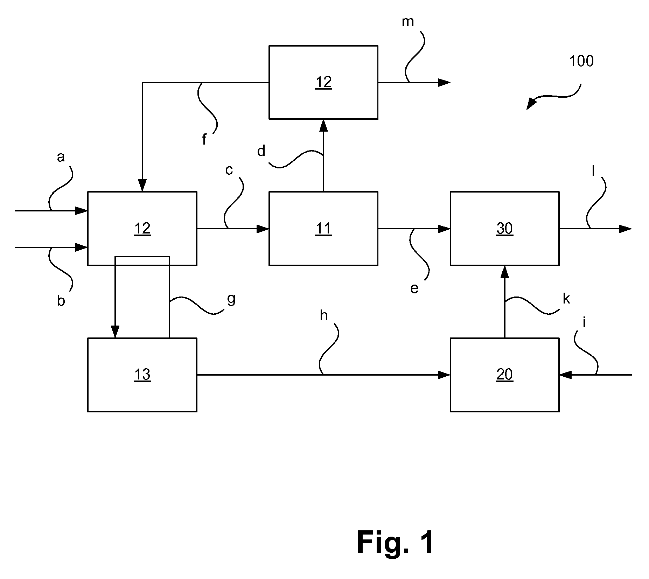

Kurze Beschreibung der Zeichnung
- Figur 1: zeigt ein Verfahren gemäß einer Ausführungsform der Erfindung in Form eines schematischen Ablaufplans.

### Ausführliche Beschreibung der Zeichnung

In Figur 1 ist ein Verfahren gemäß einer Ausführungsform der Erfindung in Form eines schematischen Ablaufplans veranschaulicht uns insgesamt mit 100 bezeichnet.

Das Verfahren 100 umfasst einen Methankopplungsschritt 10, einen Meerwasserentsalzungsschritt 20 und einen weiteren Aufbereitungsschritt 30. Dem Methankopplungsschritt 10 werden geeignete Einsatzströme, beispielsweise ein methanreicher Strom a und ein sauerstoffreicher Strom b, zugeführt. Verfahrensschritte zur Bereitstellung der Einsatzströme a und b sind der Übersichtlichkeit halber nicht gezeigt. Beispielsweise kann zur Bereitstellung des sauerstoffreichen Stroms b eine Luftzerlegungsanlage vorgesehen sein.

Aus einem Abstrom c des Methankopplungsschritts 10 können in einem oder in mehreren Fraktionierungsschritten 11 zumindest ein kohlenwasserstoffreicher Strom d sowie ein wasserreicher Strom e gebildet werden. Der zumindest eine kohlenwasserstoffreiche Strom d kann dabei beispielsweise einem oder mehreren weiteren Fraktionierungsschritten 12 zugeführt werden, in denen auch beispielsweise ein oder mehrere Recycleströme f gebildet und in den Methankopplungsschritt 10 zurückgeführt werden können. An dieser Stelle können auch eine oder mehrere Produktströme m bereitgestellt werden.

In der in Figur 1 veranschaulichten Ausführungsform der vorliegenden Erfindung wird der wasserreiche Strom e dem weiteren Aufbereitungsschritt 30 zugeführt, in dem z.B. in dem wasserreichen Strom e noch enthaltene Nebenprodukte der Methankopplung und/oder Restverunreinigungen aus dem Meerwasser entfernt werden können.

Mittels eines dem Methankopplungsschritt 10 zugeordneten Dampferzeugungsschritts 13 mit einem hier stark vereinfacht veranschaulichten Wasserkreislauf bzw. Dampfsystem g wird im dargestellten Beispiel ein Niederdruckdampfstrom h gebildet. Der Niederdruckdampfstrom h wird dem Meerwasserentsalzungsschritt 20 als Energieträger zugeführt, der, wie erwähnt, beispielsweise eine gestufte Destillation bei reduziertem Druck umfasst. Dem Meerwasserentsalzungsschritt 20 wird ferner Meerwasser in Form eines Einsatzstroms i zugeführt. Entsalztes Wasser wird als Strom k zur weiteren Aufbereitung dem weiteren Aufbereitungsschritt 30 zugeführt. Das entsalzte Wasser des Stroms k, oder ein Teil davon, kann jedoch auch aus dem Verfahren ausgeführt werden. In dem weiteren Aufbereitungsschritt 30 wird in der dargestellten Ausführungsform der Erfindung aus dem Wasser des wasserreichen Stroms e und dem entsalzten Meerwasser des Stroms k ein Strom I gebildet, der spezifikationsgerechtes Trink-, Bewässerungs- und/oder Betriebswasser umfasst.

## Patentansprüche

1. Verfahren (100) zur Gewinnung von Trink-, Bewässerungs- und/oder Betriebswasser mittels eines oder mehrerer Aufbereitungsschritte (20, 30), **dadurch gekennzeichnet, dass** ein Methankopplungschritt (10) durchgeführt wird, in dem Methan mit Sauerstoff unter Bildung von Reaktionswasser und Freisetzung von Reaktionswärme katalytisch umgesetzt wird, und dass zumindest ein Teil des Reaktionswassers und/oder zumindest ein Teil der Reaktionswärme in dem oder den Aufbereitungsschritten (20, 30) eingesetzt wird.

2. Verfahren (100) nach Anspruch 1, wobei mehrere Aufbereitungschritte (20, 30) durchgeführt werden, die einen ersten Aufbereitungsschritt (20) und einen zweiten Aufbereitungsschritt (30) umfassen.

3. Verfahren (100) nach Anspruch 2, wobei in dem ersten Aufbereitungsschritt (20) Meerwasser unter Einsatz der Reaktionswärme entsalzt wird.

4. Verfahren (100) nach Anspruch 3, wobei die Entsalzung in dem ersten Aufbereitungsschritt (20) eine gestufte Destillation bei reduziertem Druck umfasst.

5. Verfahren (100) nach Anspruch 4, wobei unter Verwendung der Reaktionswärme Niederdruckdampf gebildet und bei der Entsalzung eingesetzt wird.

6. Verfahren (100) nach einem der Ansprüche 3 bis 5, wobei in dem zweiten Aufbereitungsschritt (30) das Meerwasser, das zuvor in dem ersten Aufbereitungsschritt (20) entsalzt wurde, nachbehandelt wird.

7. Verfahren (100) nach Anspruch 6, bei dem dem ersten und/oder dem zweiten Aufbereitungsschritt (30) ferner das Reaktionswasser oder ein Teil hiervon zugeführt wird.

8. Verfahren (100) Anspruch 1, bei dem der oder die Aufbereitungsschritte (20, 30) einen Nebenproduktentfernungsschritt (30) umfassen, dem das Reaktionswasser zugeführt wird.

9. Verfahren nach einem der Ansprüche 2 bis 7 oder nach Anspruch 8, bei dem der zweite Aufbereitungsschritt (30) oder der Kohlenwasserstoffentfernungsschritt (30) einen adsorptiven, einen desorptiven und/oder einen osmotischen Reinigungsschritt umfasst.

10. Verfahren nach Anspruch 9, bei dem der zweite Aufbereitungsschritt (30) oder der Kohlenwasserstoffentfernungsschritt (30) unter Verwendung von Aktivkohle und/oder einer Umkehrosmoseeinrichtung durchgeführt wird.

11. Anlage zur Gewinnung von Gewinnung von Trink-, Bewässerungs- und/oder Betriebswasser mit einer oder mehreren Aufbereitungseinheiten, **gekennzeichnet durch** eine Methankopplungseinheit, die dafür eingerichtet ist Methan mit Sauerstoff unter Bildung von Reaktionswasser und Freisetzung von Reaktionswärme umzusetzen, und ferner **gekennzeichnet durch** Mittel, die dafür eingerichtet sind, zumindest ein Teil des Reaktionswassers und/oder zumindest ein Teil der Reaktionswärme der oder den Aufbereitungseinheiten zuzuführen.

12. Anlage nach Anspruch 11, mit einer Niederdruckdampferzeugungseinheit, die dafür eingerichtet ist, unter Verwendung der Reaktionswärme Dampf zu erzeugen und den Dampf der oder den Aufbereitungseinheiten zuzuführen.

13. Anlage nach Anspruch 11 oder 12, die zur Durchführung eines Verfahrens nach einem der Ansprüche 1 bis 10 eingerichtet ist.
